# EUROPEAN PATENT APPLICATION

(11) **EP 4 265 223 A1**
(43) Date of publication of application: **25.10.2023**
(21) Application number: 21905286.7
(22) Date of filing: 21.10.2021
(51) Int. Cl.: A61F 2/24, A61F 2/966

(54) **DELIVERY DEVICE AND DELIVERY SYSTEM**

(30) Priority: 18.12.2020 CN 202011507023
(71) Applicant: Shenzhen Lifevalve Medical Scientific Co., Ltd., Shenzhen, Guangdong 518000 (CN)
(72) Inventor: PENG, Feng, Shenzhen, Guangdong 518000 (CN)
(74) Representative: Prinz & Partner mbB
(86) International application number: PCT/CN2021/125221
(87) International publication number: WO 2022/127357

(57) **Abstract**

A delivery device (100) and a delivery system (300) are provided. The delivery device (100) includes a catheter assembly (20) and a control mechanism (10); the catheter assembly (20) comprises an outer sheath catheter (21) and a sheath core (22), the control mechanism (10) can control the outer sheath catheter (21) to move, a TIP head (23) is provided at a distal end of the sheath core (22), the control mechanism (10) comprises a fixing assembly (11) and a moving assembly (12), the moving assembly (12) is arranged close to a proximal end of the fixed assembly (11); a proximal end portion of the outer sheath catheter (21) passes through the fixing assembly (11) and then is connected to the moving assembly (12), and under the control of the moving assembly (12), the outer sheath catheter (21) can move relative to the fixed assembly (11). When the end surface of a distal end of the outer sheath catheter (21) abuts against the end surface of a proximal end of the TIP head (23), the end surface of a distal end of the moving assembly (12) is separated from the end surface of the proximal end of the fixing assembly (11). Thus, a compressible stroke is provided for the outer sheath catheter (21), solving the problem that the distal end of the outer sheath catheter (21) loaded with an implant (200) cannot be closed with the TIP head (23) due to shortening of the outer sheath catheter (21) or lengthening of the sheath core (22) when the outer sheath catheter (21) of the delivery device (100) is loaded with the implant (200).

## Description

### Technical Field

The present disclosure relates to the field of medical instruments, and more particularly relates to a delivery device and a delivery system.

### Background Art

A human heart is divided into four chambers. Each chamber has its own "exit". There are four types of valves (mitral valve, aortic valve, pulmonary valve and tricuspid valve) which ensure that the blood pumped by the heart flows in the cardiovascular system in a specified direction. The aortic valve is between the left ventricle and the ascending aorta, and plays a role of ensuring that the blood flow of the left ventricle can flow into the aortic vessel in one direction, not the other way around. When the aortic valve is opened, the high-pressure blood in the ventricle flows into the aorta through the aortic valve, and then the valve is closed. At this time, the blood in the aorta cannot return to the left ventricle through the aortic valve. However, various heart diseases or degenerative diseases will lead to partial valve dysfunction, such as valve calcification and aortic valve insufficiency. Such diseases will affect the normal work of the heart, causing gradual weakness or endangering the life of the patient.

For the absence of the function of the aortic valve, there are many methods and devices for treating the aortic valve dysfunction, such as traditional valve replacement, which is considered as "heart opening" surgery. However, such surgery is not suitable for all patients due to complex operation and significant trauma. In addition, interventional aortic valve replacement has been carefully studied. In such technologies, a self-expanding prosthetic valve is generally mounted at a tail end of a flexible catheter in a compressed state and is pushed via the patient's blood vessel or body until the prosthetic valve reaches an implantation site. The prosthetic valve then expands to its functional size at the location of a defective natural aortic valve.

In transcatheter aortic valve replacement, a usual surgical path is to push a delivery system loaded with a prosthetic valve to a diseased valve through the aorta from the femoral artery. At this time, the delivery system needs to pass through the entire aortic arch. In order to make the delivery system pass through a curved blood vessel at the arch more easily, the usual practice is to reduce an outer diameter of a sheath catheter as much as possible. However, this method often leads to a greater compression deformation of a valve when the valve is received into the sheath catheter, so that the loading force will also increase. The sheath catheter/sheath core of the delivery system is easily lengthened or shortened during loading, and the delivery system cannot be closed after being loaded, causing a difficulty or failure during a procedure.

### Summary of the Disclosure

The present disclosure provides a delivery device. The delivery device includes a catheter assembly and a control mechanism; the catheter assembly includes an outer sheath catheter and a sheath core; the control mechanism can control the sheath catheter to move; a TIP head is arranged at a distal end of the sheath core; the control mechanism includes a fixed assembly and a moving assembly; the moving assembly is arranged close to a proximal end of the fixed assembly; a proximal end portion of the sheath catheter passes through the fixed assembly and then is connected to the moving assembly, and under the control of the moving assembly, the sheath catheter can move relative to the fixed assembly; and when the end surface of a distal end of the sheath catheter abuts against the end surface of a proximal end of the TIP head, the end surface of a distal end of the moving assembly is separated from the end surface of the proximal end of the fixed assembly.

In one embodiment, a proximal end portion of the fixed assembly includes a receiving portion with an opening facing the proximal end, and the receiving portion can receive a distal end portion of the moving assembly; or a distal end portion of the moving assembly includes a receiving portion with an opening facing the distal end, and the receiving portion can receive the proximal end portion of the fixed assembly.

In one embodiment, a stop position is arranged on an outer surface of the distal end portion of the moving assembly or an outer surface of the proximal end portion of the fixed assembly; and when an end surface of the receiving portion close to the stop position abuts against the stop position, the moving assembly does not axially move relative to the fixed assembly.

In one embodiment, the moving assembly includes an elastic member and a moving member; the proximal end of the fixed assembly includes a receiving portion with an opening facing the proximal end or the distal end of the moving assembly includes a receiving portion with an opening facing the distal end; the elastic member is arranged in an inner cavity of the receiving portion; the moving member is arranged close to the elastic member; and the moving member is at least partially received in the receiving portion and is movably connected with the receiving portion, so that the elastic member is compressed.

In one embodiment, a first limiting member is arranged on an inner wall of the receiving portion; a second limiting member is arranged on the moving member; and when the first limiting member and the second limiting member are in contact, the moving member is restrained from being away from the receiving portion.

In one embodiment, the moving member can move towards a bottom surface of the inner cavity of the receiving portion, compress the elastic member, and separate the first limiting member from the second limiting member.

The present disclosure further provides a delivery system, including any one of the above delivery devices, and an implant. The implant can be loaded in the outer sheath catheter.

In one embodiment, after the implant is loaded into the outer sheath catheter, the end surface of the distal end of the outer sheath catheter abuts against the end surface of the proximal end of the TIP head, and a distance between the end surface of the distal end of the moving assembly and the end surface of the proximal end of the fixed assembly is shorter than a distance between the end surface of the distal end of the moving assembly and the end surface of the proximal end of the fixed assembly before the implant is loaded.

In one embodiment, the fixed assembly further includes a moving track; the moving assembly includes an operating portion; the operating portion has an inner cavity; the moving track passes through the inner cavity of the operating portion; and the operating portion can axially move along the moving track relative to the fixed assembly.

In one embodiment, when the first limiting member and the second limiting member are separated, the operating portion can further move towards the distal end.

According to the delivery device and the delivery system of the present disclosure, by the arrangement of the moving assembly and the fixed assembly, before the implant is loaded, the end surface of the distal end of the moving assembly is separated from the end surface of the proximal end of the fixed assembly, which provides a compressible stroke for the outer sheath catheter, solving the problem that the distal end of the outer sheath catheter loaded with an implant cannot be closed with the TIP head due to shortening of the outer sheath catheter or lengthening of the sheath core when the outer sheath catheter of the delivery device is loaded with the implant.

### Brief Description of the Drawings

Fig. 1 is a schematic diagram of an entire structure of a delivery device of one embodiment of the present disclosure;
Fig. 2 is a partially structural enlarged diagram of the delivery device shown in Fig. 1;
Fig. 3 is a partially structural exploded diagram of a control mechanism of the delivery device shown in Fig. 1;
Fig. 4 is a combined diagram of partial structures of the control mechanism shown in Fig. 3;
Fig. 5 is a sectional diagram of partial structures of the control mechanism shown in Fig. 4;
Fig. 6 is an exploded sectional diagram of partial structures of the control mechanism shown in Fig. 5;
Fig. 7.1 is a partially structural sectional diagram of the delivery device shown in Fig. 1;
Fig. 7.2 is an enlarged diagram of partial structures of Fig. 7.1;
Fig. 8 is a schematic structural diagram of a delivery device in a delivery system of one embodiment of the present disclosure;
Fig. 9 is a schematic structural diagram of a delivery system of one embodiment of the present disclosure, illustrating a process state of loading an implant into the delivery device shown in Fig. 8;
Fig. 10 is a schematic structural diagram after the loading of the delivery system shown in Fig. 9 is completed;
Fig. 11 is a schematic diagram of a sectional structure of a control mechanism of a delivery device of another embodiment of the present disclosure;
Fig. 12 is a partially structural sectional diagram of the control mechanism of the delivery device shown in Fig. 11;
Fig. 13 is a schematic diagram of a sectional structure of a control mechanism of a delivery device of still another embodiment of the present disclosure; and
Fig. 14 is a schematic diagram of a sectional structure of a control mechanism of a delivery device of yet another embodiment of the present disclosure.

### Detailed Description of the Disclosure

In order to better understand the technical solutions and effective effects of the present disclosure, the technical solutions of the present disclosure are exemplified in combination with the accompanying drawings. The following specific embodiments are only part of the embodiments, and not intended to limit the present disclosure.

The meanings of the technical terms used in the present disclosure are all general meanings understood by those skilled in the art unless otherwise stated.

In the field of medical instruments, it is defined that an end close to an operator is a proximal end, and an end far away from the operator is a distal end, so as to describe the proximal end and the distal end of a certain component and a relative positional relationship between components.

### Embodiment I

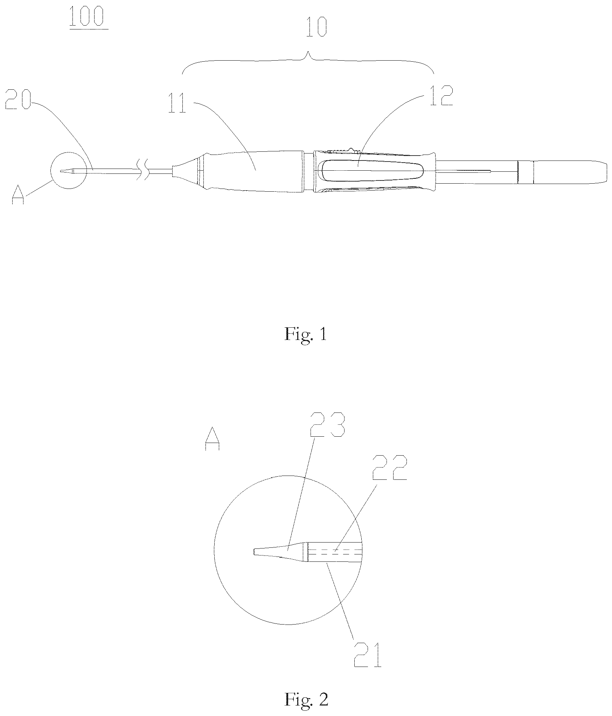

As shown in Fig. 1 and Fig. 2, a delivery device 100 according to this embodiment includes a control mechanism 10 and a catheter assembly 20. An operator can control the catheter assembly 20 to move through the control mechanism 10. The catheter assembly 20 includes an outer sheath catheter 21 and a sheath core 22. The outer sheath catheter 21 is a hollow tube body with an inner cavity, and the sheath core 22 is threaded through the inner cavity of the outer sheath catheter 21. A TIP head 23 is arranged at a distal end of the sheath core 22. The TIP head 23 is a tapered structure with an outer diameter gradually decreasing toward the distal end, which is made of a polymer material to guide the delivery device to move in a blood vessel. The outer sheath catheter 21 can be close to or far from the TIP head 23 under the control of the control mechanism 10. When the end surface of a distal end of the outer sheath catheter 21 abuts against and contacts the end surface of a proximal end of the TIP head 23, the outer sheath catheter 21 is blocked by the TIP head 23 and cannot continue to move towards the distal end.

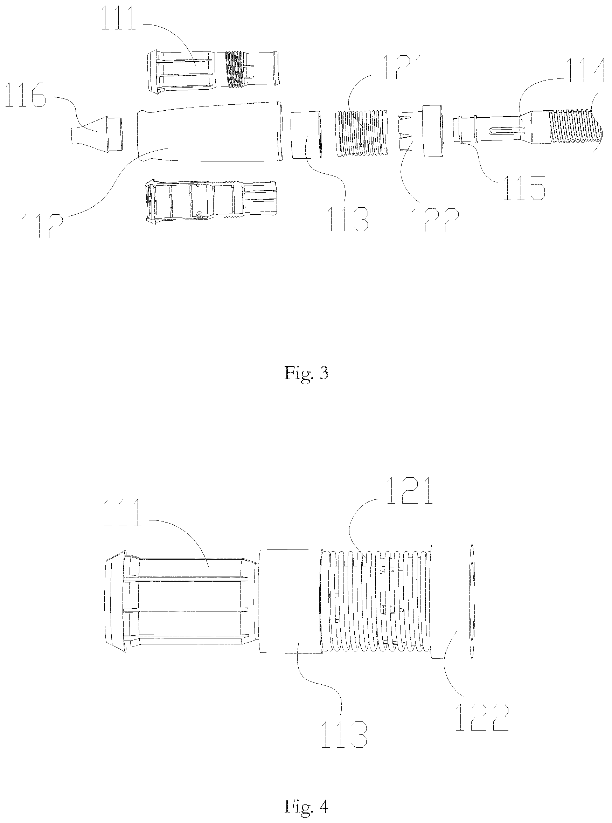

As shown in Fig. 1 and Fig. 3, the control mechanism 10 includes a fixed assembly 11 and a moving assembly 12. The fixed assembly 11 and the moving assembly 12 are arranged adjacent to each other, and the moving assembly 12 is arranged close to a proximal end of the fixed assembly 11. A proximal end portion of the outer sheath catheter 21 passes through the fixed assembly 11, then is connected with the moving assembly 12, and can move relative to the fixed assembly 11 and the sheath core 22 under the control of the moving assembly 12. When the end surface of the distal end of the outer sheath catheter 22 abuts against the end surface of the proximal end of the TIP head 23, the end surface of the distal end of the moving assembly 12 is separated from the end surface of the proximal end of the fixed assembly 11; that is, there is no contact.

The fixed assembly 11 includes a handheld portion 112 and a mounting seat 111. Each of the handheld portion 112 and the mounting seat includes a hollow shell with openings in both ends, and the mounting seat 111 is arranged in a cavity of the handheld portion 112 and is fixed relative to the handheld portion 112. The mounting seat 111 and the handheld portion 112 can be connected in various ways. For example, a groove can be arranged on an outer surface of the mounting seat 111. Correspondingly, a rib matched with the groove is arranged on an inner surface of the handheld portion 112. Relative fixation between the mounting seat 111 and the handheld portion 112 is realized by clamping of the rib and the groove. It can be understood that the handheld portion can be formed by splicing two shells, or can be integrally machined. Similarly, the mounting seat 111 can also be formed by splicing at least two shells, or can be integrally machined.

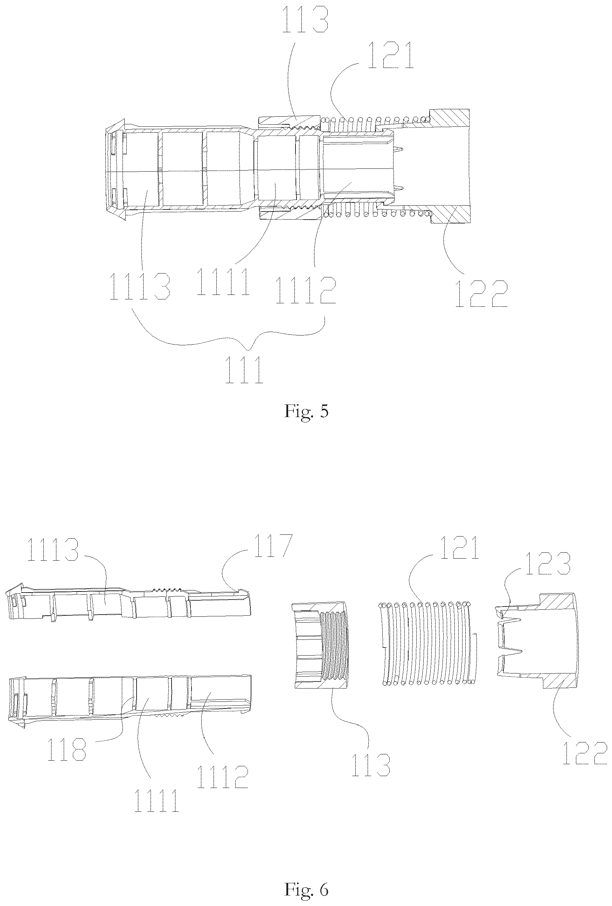

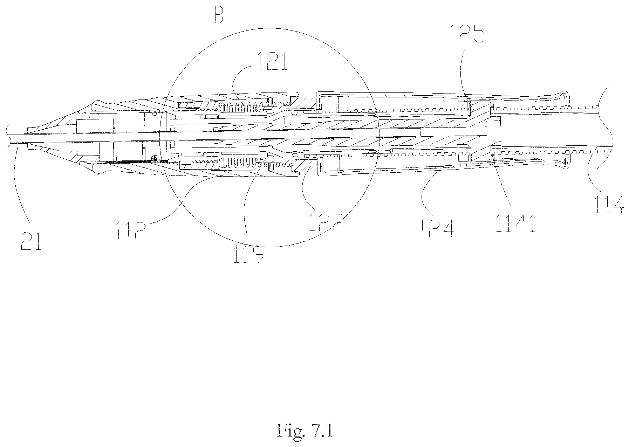

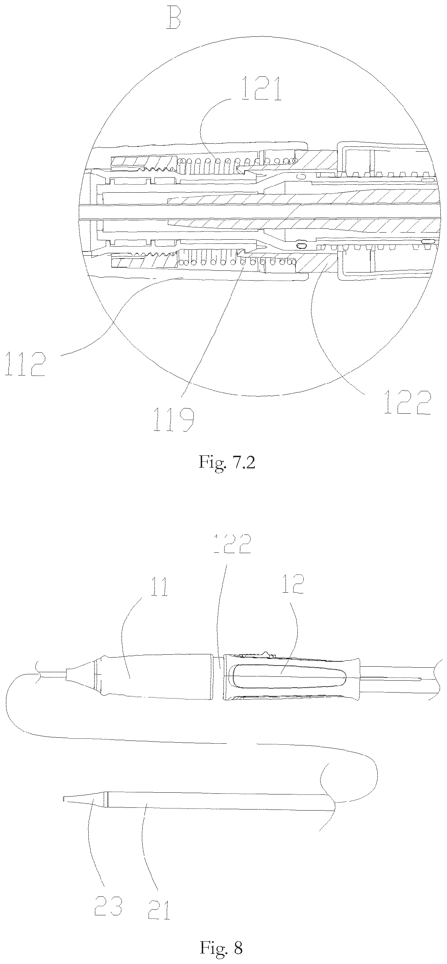

As shown in Fig. 4 and Fig. 5, the mounting seat 111 includes a middle portion 1111 with a reduced outer diameter, a proximal end portion 1112 arranged at a proximal end of the middle portion 1111, and a distal end portion 1113 arranged at a distal end of the middle portion 1111. The outer diameter of the middle portion 1111 and an outer diameter of the proximal end portion 1112 are both less than an outer diameter of the distal end portion 1113, and the outer diameter of the proximal end portion 1112 is less than the outer diameter of the middle portion 1111. In other embodiments, the outer diameter of the proximal end portion 1112 may be slightly greater than the outer diameter of the middle portion 1111, but still less than the outer diameter of the distal end portion 1113. Thus, a receiving portion 119 (referring to Fig. 7.1 and Fig. 7.2) with an opening facing the proximal end is formed between an outer surface of the mounting seat 111 and an inner surface of the handheld portion 112, and a bottom surface of the receiving portion 119 is formed at the distal end of the middle portion 1111.

As shown in Fig. 3 to Fig. 6, the moving assembly 12 includes an elastic member 121 and a moving member 122. The elastic member 121 is arranged in the inner cavity of the receiving portion 119. The moving member 122 is arranged close to a proximal end of the elastic member 121, and the moving member 122 is partially received in the receiving portion 119 and is movably connected with the receiving portion 119, so that the elastic member 121 is compressed.

Specifically, the fixed assembly 11 also includes a base 113. The base 113 surrounds the middle portion 1111 of the mounting seat 111. In this embodiment, the base 113 is of a hollow annular structure. An inner wall of the base 113 is provided with an internal thread, and the middle portion 1111 is provided with an external thread, so that the base 113 is in threaded connection with the middle portion 1111, and the base 113 is fixed on the mounting seat 111. The base 113 is accommodated in the receiving portion 119 between the mounting seat 111 and the handheld portion 112. The elastic member 121 is a spring, which surrounds a periphery of the proximal end portion 1112 of the mounting seat 111, and the end surface of the distal end of the elastic member 121 abuts against the end surface of the proximal end of the base 113.

As shown in Fig. 5 and Fig. 6, the moving member 122 is of a hollow structure with openings in both ends, and the openings in both ends are communicated with each other. An outer surface of the moving member 122 is provided with a step, so that the outer diameter of the proximal end portion is greater than the outer diameter of the distal end portion, and the outer diameter of the proximal end portion is less than an inner diameter of the receiving portion 119. A distal end portion of the moving member 122 is threaded in the elastic member 121, so that the end surface of the proximal end of the elastic member 121 abuts against the step surface of the outer surface of the moving member 122. In order to realize a continuous pressure exerted by the moving member 122 on the elastic member 121, the inner surface of the receiving portion 119 is provided with a first limiting member 117, and the inner surface of the distal end portion of the moving member 122 is provided with a second limiting member 123. In this embodiment, both the first limiting member 117 and the second limiting member 123 are slightly hook-like protrusions. In a natural state, the first limiting member 117 is in contact with the second limiting member 123. When the moving member 122 moves towards the distal end under the action of a force towards the distal end, the first limiting member 117 and the second limiting member 123 are far away from each other, and the elastic member 121 is further compressed. When the force towards the distal end is removed, the moving member 122 moves towards the proximal end under the action of the elastic member 121, and the second limiting member 123 also moves towards the proximal end. When the second limiting member 123 is in contact with the first limiting member 117, the moving member 122 stops moving, thus restraining the moving member 122 from being far away from the receiving portion 119, and making the elastic member 121 always compressed. In this embodiment, the first limiting member 117 is arranged at the proximal end of the outer surface of the proximal end portion 1112 of the mounting seat 111, and is of a hook-like structure extending towards the distal end. It can be understood that the first limiting member and the second limiting member can be arranged continuously along a circumferential direction, or can be arranged in breakpoint distribution.

It can be understood that in other embodiments, the base 113 may not be provided. At this time, the distal end of the elastic member 121 can be in direct contact with the bottom surface of the receiving portion 119, and always abuts against the bottom surface of the receiving portion 119 under the action of the moving member 122.

It can also be understood that in other embodiments, the receiving portion can also be arranged on the moving assembly. Specifically, the receiving portion can be provided with the distal end of the moving assembly, and has its opening facing the distal end and receiving the elastic member. The moving member is also partially limited in the receiving portion. The specific structure of this portion is the same as that of this embodiment, as long as the corresponding structures replace those on the moving assembly.

As shown in Fig. 3, Fig. 7.1 and Fig. 7.2, the delivery device also includes a protective sleeve 116 and a moving track 114. The protective sleeve 116 is arranged at the distal end of the fixed assembly 11. Specifically, the protective sleeve 116 is clamped on an opening in the distal end of the handheld portion 112. The protective sleeve 116 is roughly of a tapered structure, with an outer diameter gradually decreasing from the proximal end to the distal end, thereby protecting the outer sheath catheter 21 and buffering the stress from the distal end of the handheld portion 112 on the outer sheath catheter 21. Preferably, the protective sleeve 116 may be made of a material with hardness that is slightly lower than that of a material for making the handheld portion 112.

In this embodiment, a portion of the moving track 114 close to the distal end is provided with an annular ridge 115. The annular ridge 115 is clamped with the groove 118 on the inner surface of the mounting seat 111, so that the moving track 114 is fixed relative to the mounting seat 111 and the handheld portion 112. The moving track 114 is exposed to the proximal end of the fixed assembly 11 except that its distal end is arranged inside the fixed assembly 11.

The moving assembly also includes an operating portion 124. The operating portion 124 includes an inner cavity, and both ends are provided with openings. The inner cavity of the operating portion 124 is communicated with the openings at both ends. The inner diameter of a distal end of the inner cavity of the operating portion 124 is less than the outer diameter of the proximal end portion of the moving member 122, and the end surface of a distal end of the operating portion 124 may be far away from the end surface of the proximal end of the moving member 122 or abuts against the end surface of the proximal end of the moving member 122.

The operating portion 124 sleeves the above portion of the moving track 114 exposed to the proximal end of the fixed assembly 11, and the inner surface of the operating portion 124 is in direct contact with the moving track 114 The outer surface of the moving track 114 exposed to the proximal end of the fixed assembly 11 is provided with a partial external thread, and an internal thread matched with the external thread on the outer surface of the moving track 114 is arranged inside the operating portion 124. Thus, the operating portion 124 can be rotated to circumferentially rotate and axially move on the moving track 114, and be close to or far from the fixed assembly 11. From the perspective of ergonomics, in order to make the operator comfortable during operation, it is preferred that the outer diameter of the operating portion is naturally transitioned when the operating portion is in contact with the handheld portion. When the end surface of the distal end of the operating portion is in contact with the end surface of the proximal end of the handheld portion, the operating portion cannot continue to move toward the handheld portion.

Both ends of the moving track 114 are provided with openings, and an inner cavity communicated with the openings at both ends is arranged inside. A side wall opening 1141 communicated with the inner cavity is also formed in the outer surface of the moving track 114. The side wall opening 1141 extends on the outer surface of the moving track 114 along a longitudinal direction of the moving track 114, but an extending length does not exceed the length of the moving track 114. The moving assembly 12 also includes a connector 125. The connector 125 is arranged in the inner cavity of the moving track 114, and a proximal end portion of the connector 125 protrudes from the side wall opening 1141, so as to be clamped on the operating portion 124 to achieve relative fixation with the operating portion 124. Both ends of the connector 125 are also provided with openings, and an inner cavity communicated with the openings at both ends is arranged inside. The proximal end portion of the outer sheath catheter 21 is connected to the inner cavity of the connector 125 and is connected with the connector 125 via welding, bonding or threaded connection. Therefore, when the operating portion 124 moves on the moving track 114, it will drive the connector 125 to move in the side wall opening 1141 along the lengthwise direction of the moving track 114, thus driving the outer sheath catheter 21 to move through the connector 125.

In the natural state, the end surface of the distal end of the outer sheath catheter 21 abuts against the end surface of the proximal end of the TIP head 23; the end surface of the distal end of the operating portion 124 abuts against the end surface of the proximal end of the moving member 122; and the end surface of the distal end of the operating portion 124 is separated from the end surface of the proximal end of the handheld portion 112. The elastic member 121 can still be compressed through the moving member 122 when the operating portion 124 moves towards the distal end, but the TIP head 23 restrains the outer sheath catheter 21 from continuing to move towards the distal end, so that without damaging the outer sheath catheter, the operating portion 124 cannot continue to move towards the distal end and the elastic member 121 cannot be continuously compressed. It can be understood that in other embodiments, in the natural state, the end surface of the distal end of the operating portion can also be separated from the end surface of the proximal end of the moving member. However, in any case, the end surface of the distal end of the operating portion is separated from the end surface of the proximal end of the handheld portion, so that when the implant is loaded, the operating portion can still move toward the distal end after the outer sheath catheter is shortened or the sheath core is lengthened, so as to drive the outer sheath catheter to continue to move towards the distal end.

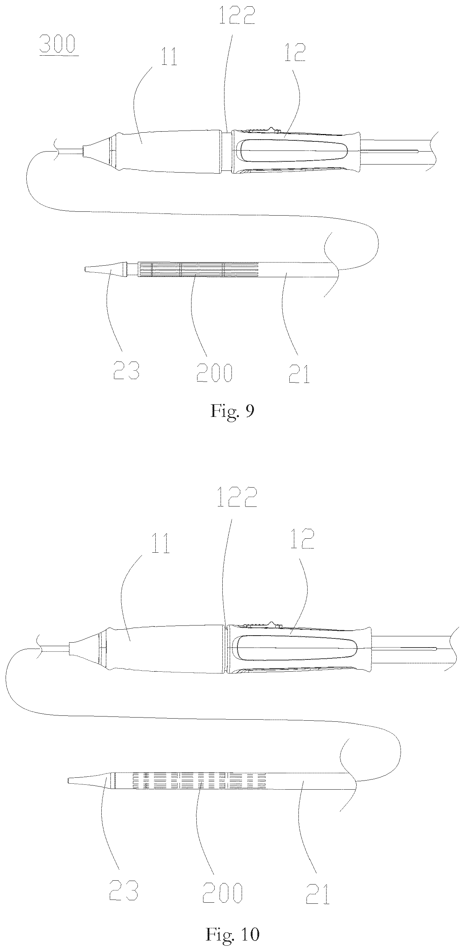

As shown in Fig. 8 to Fig. 10, the present disclosure also provides a delivery system 300. The delivery system 300 includes a delivery device 100 of the present disclosure, and an implant 200. The implant 200 can be loaded into the outer sheath catheter 21 of the delivery system 100, and the implant 200 can be delivered into an organism through the delivery system 100. Before the implant 200 is loaded to the delivery device 100 (that is, when the delivery device 100 is in the natural state), the end surface of the distal end of the outer sheath catheter 21 of the delivery device 100 abuts against the end surface of the proximal end of the TIP head 23, and the end surface of the distal end of the moving assembly 12 is separated from the end surface of the proximal end of the fixed assembly 11, so that the moving member 122 is partially exposed from the handheld portion 112. It should be understood that at this time, although the moving member is located in the inner cavity of the handheld portion, the end surface of the distal end of the moving member is also separated from the end surface of the proximal end of the handheld portion. When the implant 200 is loaded, after the implant 200 is fixed on the delivery device 100, the outer sheath catheter 21 moves towards the distal end by operating the moving assembly 12, so that the end surface of the distal end of the outer sheath catheter 21 abuts against the end surface of the proximal end of the TIP head 23, so as to complete the loading of the implant 200. During the loading, due to a radial expansion force of the implant 200 itself, if a loading force is extremely high, and the resistance of the implant 200 on the outer sheath catheter 21 towards the proximal end is also higher. In the field of medical instruments, the outer sheath catheter is usually made of a polymer material, so that the outer sheath catheter 21 will be shortened under the action of the loading resistance. In the natural state, the moving assembly of the delivery device is separated from the fixed assembly, and a compressible stroke is reserved for the further movement of the outer sheath catheter after the outer sheath catheter is shortened. Therefore, even if the operating portion 124 abuts against the moving member 122, the outer sheath catheter 21 and the TIP head 23 are still separated, and the operating portion 124 can still continue to move towards the distal end to push the moving member 122 to compress the elastic member 121, so that the outer sheath catheter 21 continues to move towards the distal end until the end surface of the distal end of the outer sheath catheter 21 abuts against the end surface of the proximal end of the TIP head 23.

In order to better represent the positional relationship among the distal end of the outer sheath catheter, the TIP head and the implant, the distal end portion of the delivery system of Fig. 8 to Fig. 10 has been enlarged.

It can be understood that the outer sheath catheter may or may not be shortened due to the various sizes of the implant. When the outer sheath catheter is shortened, the shortening size will also vary depending on the size of the implant. Therefore, after the moving member further compresses the elastic member to compensate for the shortening distance of the outer sheath catheter to complete the loading of the implant, the operating portion and the handheld portion are not necessarily in contact, but they are possibly separated.

Therefore, in this embodiment, since the elastic member and the moving member are arranged on the moving assembly, a compressible stroke is provided for the outer sheath catheter, solving the problem that the distal end of the outer sheath catheter loaded with an implant cannot be closed with the TIP head due to the shortening of the outer sheath catheter or lengthening of the sheath core when the outer sheath catheter of the delivery device is loaded with the implant.

### Embodiment II

The delivery device of this embodiment is basically the same as the delivery device of Embodiment I, and a difference only lies in the control mechanism.

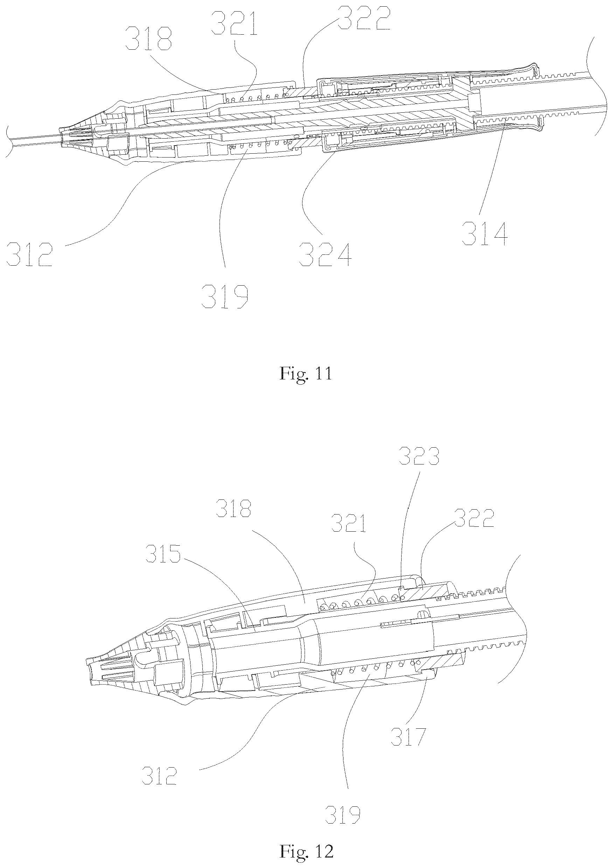

As shown in Fig. 11 and Fig. 12, the control mechanism of this embodiment is not provided with the mounting seat. A plurality of ridges 318 are arranged on the inner wall of the handheld portion 312. The annular ridges 315 on the distal end portion of the moving track 314 is in limiting clamping with the ridges 318 on the handheld portion 312, so that the moving track 314 is fixed through the handheld portion 312. A receiving portion 319 with an opening facing the proximal end is formed between the inner cavity of the handheld portion 312 and the distal end portion of the moving track 314. The elastic member 321 is arranged in the receiving portion 319, and the distal end of the elastic member 321 abuts against the ridges 318 on the inner wall of the handheld portion 312, thus restraining the distal end of the elastic member 321 from continuing to move towards the distal end. The distal end portion of the moving member 322 is movably received in the receiving portion 319, and the proximal end of the moving member 322 can protrude from the proximal end of the receiving portion 319. In this embodiment, the first limiting member 317 is arranged on the inner wall of the handheld portion 312 (i.e. the inner wall of the receiving portion), and the second limiting member 323 is arranged on the outer surface of the moving member 322, so that the first limiting member 317 and the second limiting member 323 can be far from or close to each other under the action of an external force. It can be understood that the first limiting member can be a ridge arranged on the handheld portion 312. The operating portion 324 is arranged close to the proximal end of the moving member 322, and will not be beyond the moving member when moving. The specific structure is similar to that of Embodiment I, and will not be repeatedly described.

It can be understood that the receiving portion of this embodiment can also be arranged on the moving assembly. Specifically, the receiving portion can be provided with the distal end of the handheld portion, and has its opening facing the distal end and receiving the elastic member. The moving member is also partially limited in the receiving portion. The specific structure of this portion is the same as that of this embodiment, as long as the corresponding structures replace those on the operating portion.

The operation of loading an implant into the delivery device of this embodiment and the principle of compensating for the shortening of the outer sheath catheter are the same as those of Embodiment I, and will not be repeatedly described.

According to the delivery device of this embodiment, since the elastic member and the moving member are arranged on the moving assembly, a compressible stroke is provided for the outer sheath catheter, solving the problem that the distal end of the outer sheath catheter loaded with an implant cannot be closed with the TIP head due to the shortening of the outer sheath catheter or lengthening of the sheath core when the outer sheath catheter of the delivery device is loaded with the implant. In addition, the control mechanism has a simpler structure.

### Embodiment III

The delivery device of this embodiment is basically the same as that of Embodiment I, and a difference lies in the control mechanism in which no elastic member and no moving member are provided.

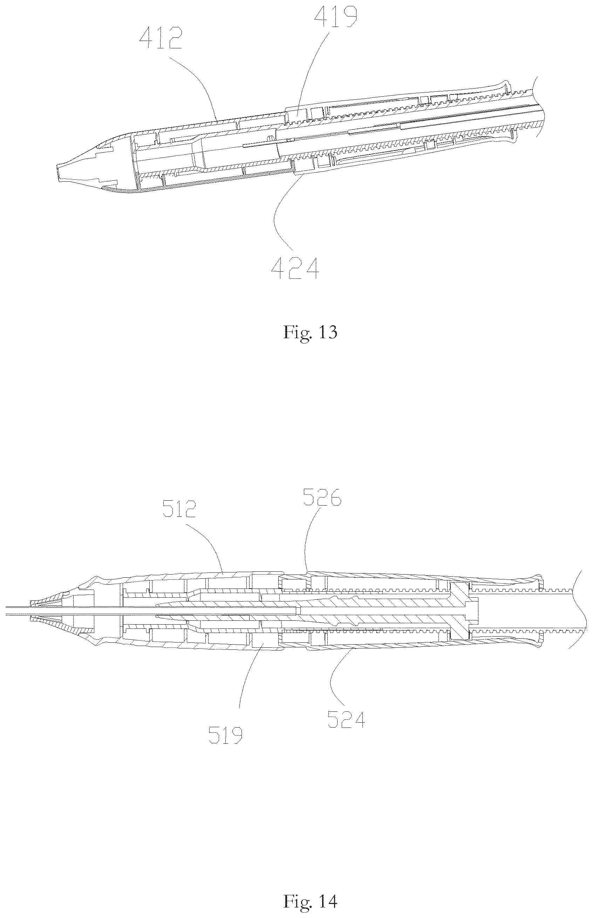

As shown in Fig. 13, the control mechanism of this embodiment includes a fixed assembly and a moving assembly. The distal end portion of the moving assembly 424 includes a receiving portion 419 with an opening facing the distal end. The receiving portion 419 can receive the proximal end portion of the fixed assembly 412, that is, the size of the inner cavity of the receiving portion 419 is greater than the outer diameter of the proximal end portion of the fixed assembly 412. Preferably, the inner diameter of the receiving portion 419 is equal to the outer diameter of the proximal end portion of the fixed assembly 412. It can be understood that the inner diameter of the receiving portion can gradually increase from the proximal end to the distal end. Correspondingly, the outer diameter of the proximal end portion of the fixed assembly gradually decreases from the distal end to the proximal end.

It can be understood that in other embodiments, the receiving portion and received portion on the control mechanism can be interchanged. As shown in Fig. 14, the proximal end portion of the fixed assembly 512 is provided with a receiving portion 519 with an opening facing the proximal end, and the receiving portion 519 can receive the distal end portion of the moving assembly 524. In addition, in order to make the outer diameter of the control mechanism have a natural transition and to restrain the maximum distance of movement of the moving assembly relative to the fixed assembly, the outer surface of the distal end portion of the moving assembly 524 is provided with a stop position 526. The stop position 526 can be a step surface formed on the outer surface of the moving assembly 524. When the end surface of the proximal end of the receiving portion 519 abuts against the stop position 526, the moving assembly 524 cannot continue to move towards the fixed assembly 512.

It can also be understood that when the receiving portion is arranged at the distal end portion of the moving assembly, the outer surface of the fixed assembly can also be provided with a stop position.

Other structures of this embodiment are similar to those of Embodiment I, and will not be repeatedly described here.

Before the delivery device of this embodiment is used to load an implant, the end surface of the proximal end of the TIP head abuts against the end surface of the distal end of the outer sheath catheter, the receiving portion 419 only receives a small part of the proximal end portion of the fixed assembly 412. When the implant is loaded and the outer sheath catheter is shortened, the moving assembly continues to move towards the distal end, so that the receiving portion receives a large part of the proximal end portion of the fixed assembly 412, so as to compensate for the shortening size of the outer sheath catheter and complete the loading of the implant.

According to the delivery device of this embodiment, since the elastic member and the moving member are arranged on the moving assembly, a compressible stroke is provided for the outer sheath catheter, solving the problem that the distal end of the outer sheath catheter loaded with an implant cannot be closed with the TIP head due to the shortening of the outer sheath catheter or lengthening of the sheath core when the outer sheath catheter of the delivery device is loaded with the implant. In addition, the control mechanism has a simpler structure.

The above embodiments are only the preferred embodiments of the present disclosure. This specification cannot enumerate all the optional embodiments in detail. Those skilled in the art can divide, combine or omit some structures of the above embodiments according to actual needs. Without changing the concept of the present disclosure, the splitting, combination and omission are all technical solutions falling within the present disclosure.

## Claims

1. A delivery device, comprising a catheter assembly and a control mechanism, wherein the catheter assembly comprises an outer sheath catheter and a sheath core; the control mechanism controls the sheath catheter to move; a TIP head is arranged at a distal end of the sheath core; the control mechanism comprises a fixed assembly and a moving assembly; the moving assembly is arranged close to a proximal end of the fixed assembly; a proximal end portion of the sheath catheter passes through the fixed assembly and then is connected to the moving assembly, and under the control of the moving assembly, the sheath catheter is configured to move relative to the fixed assembly; and when the end surface of a distal end of the sheath catheter abuts against the end surface of a proximal end of the TIP head, the end surface of a distal end of the moving assembly is separated from the end surface of the proximal end of the fixed assembly.

2. The delivery device according to claim 1, wherein a proximal end portion of the fixed assembly comprises a receiving portion with an opening facing the proximal end, and the receiving portion is configured to receive a distal end portion of the moving assembly; or a distal end portion of the moving assembly comprises a receiving portion with an opening facing the distal end, and the receiving portion is configured to receive the proximal end portion of the fixed assembly.

3. The delivery device according to claim 2, wherein a stop position is arranged on an outer surface of the distal end portion of the moving assembly or an outer surface of the proximal end portion of the fixed assembly; and when an end surface of the receiving portion close to the stop position abuts against the stop position, the moving assembly does not axially move relative to the fixed assembly.

4. The delivery device according to claim 1, wherein the moving assembly comprises an elastic member and a moving member; the proximal end of the fixed assembly comprises a receiving portion with an opening facing the proximal end or the distal end of the moving assembly comprises a receiving portion with an opening facing the distal end; the elastic member is arranged in an inner cavity of the receiving portion; the moving member is arranged close to the elastic member; and the moving member is at least partially received in the receiving portion and is movably connected with the receiving portion, so that the elastic member is compressed.

5. The delivery device according to claim 4, wherein a first limiting member is arranged on an inner wall of the receiving portion; a second limiting member is arranged on the moving member; and when the first limiting member and the second limiting member are in contact, the moving member is restrained from being away from the receiving portion.

6. The delivery device according to claim 5, wherein the moving member is configured to move towards a bottom surface of the inner cavity of the receiving portion, compress the elastic member, and separate the first limiting member from the second limiting member.

7. A delivery system, comprising the delivery device according to claims 1 to 6, and an implant, wherein the implant is configured to be loaded in the outer sheath catheter.

8. The delivery system according to claim 7, wherein after the implant is loaded into the outer sheath catheter, the end surface of the distal end of the outer sheath catheter abuts against the end surface of the proximal end of the TIP head, and a distance between the end surface of the distal end of the moving assembly and the end surface of the proximal end of the fixed assembly is shorter than a distance between the end surface of the distal end of the moving assembly and the end surface of the proximal end of the fixed assembly before the implant is loaded.

9. The delivery system according to claim 7, wherein the fixed assembly further comprises a moving track; the moving assembly comprises an operating portion; the operating portion has an inner cavity; the moving track passes through the inner cavity of the operating portion; and the operating portion is configured to axially move along the moving track relative to the fixed assembly.

10. The delivery system according to claim 9, wherein when the first limiting member and the second limiting member are separated, the operating portion is configured to further move towards the distal end.
